# EUROPEAN PATENT SPECIFICATION

(11) **EP 3 927 428 B1**
(45) Date of publication and mention of the grant of the patent: **05.03.2025**
(21) Application number: 20760077.6
(22) Date of filing: 14.02.2020
(51) Int. Cl.: A61K 31/4535, A61P 11/16, A61P 11/06, A61P 31/04

(54) **NORKETOTIFEN FOR USE IN THE TREATMENT OF RESPIRATORY DISORDERS**
NORKETOTIFEN ZUR BEHANDLUNG VON ATEMWEGSERKRANKUNGEN
NORKÉTOTIFÈNE POUR LE TRAITEMENT DES TROUBLES RESPIRATOIRES

(30) Priority: 22.02.2019 US 201962809212 P; 24.01.2020 US 202016751539
(43) Date of publication of application: 29.12.2021
(73) Proprietor: Bridge Pharma, Inc., Sarasota, FL 34242 (US)
(72) Inventor: ABERG, A. K. Gunnar, Sarasota, Florida 34242 (US); CIOFALO, Vincent, B., Branford, Connecticut 06405 (US); PUCAJ, Kresimir, 10090 Zagreb (HR)
(74) Representative: Harris, Oliver John Richard
(86) International application number: PCT/US2020/018205
(87) International publication number: WO 2020/172047

(56) References cited:
- WO-A2-2012/108635
- US-A1- 2007 191 726
- US-A1- 2010 166 804
- US-A1- 2013 045 921
- US-A1- 2015 272 941
- US-A1- 2018 072 796
- US-B1- 7 226 934
- WEBLEY WILMORE C. ET AL: "Infection-mediated asthma: etiology, mechanisms and treatment options, with focus on Chlamydia pneumoniae and macrolides", RESPIRATORY RESEARCH, vol. 18, no. 1, 19 May 2017 (2017-05-19), XP055968418, Retrieved from the Internet <URL:http://link.springer.com/content/pdf/10.1186/s12931-017-0584-z.pdf> [retrieved on 20221004], DOI: 10.1186/s12931-017-0584-z
- MIRZA SHIREEN ET AL: "COPD Guidelines: A Review of the 2018 GOLD Report", MAYO CLINIC PROCEEDINGS, vol. 93, no. 10, 1 October 2018 (2018-10-01), US, pages 1488 - 1502, XP055968426, ISSN: 0025-6196, DOI: 10.1016/j.mayocp.2018.05.026

## Description

### TECHNICAL FIELD

The embodiments disclosed herein relate to the treatment of airways and pulmonary disorders with norketotifen, specifically RS-norketotifen.

### BACKGROUND

Pulmonary disorders such as asthma and chronic obstructive pulmonary disorder (COPD) are significant pulmonary problems in humans. Asthma is an inflammatory disease of the lungs that affects all age groups of patients and is characterized by recurrent attacks (exacerbations) of breathlessness and wheezing. The global prevalence of asthma is about 360 million patients with an annual asthma-related death rate of about 400,000 patients. Asthma-related deaths occur in connection with exacerbations ("attacks") of the disease. COPD is an umbrella term that is used to cover certain inflammatory lung diseases such as "emphysema" and "chronic bronchitis". The estimated global prevalence of COPD is 175 million patients and the annual COPD-related death rate has been estimated at 3.2 million patients. COPD-related deaths occur in connection with exacerbations of the disease.

In humans, both asthma and COPD are currently treated with inhaled anti-inflammatory corticosteroids, inhaled bronchodilators, and combinations thereof. The steroids are usually administered directly into the lungs by use of various types of inhalers. Currently, no potent inflammatory drugs are available that are free from the adverse immune-suppressive effects. Even the anti-inflammatory monoclonal antibodies potently express adverse suppression of the immune system.

US2015272941A1 discloses the use of norketotifen in the treatment of a chronic, inflammatory pulmonary condition, including asthma and COPD.

What is needed for the treatment of pulmonary inflammatory conditions are non-steroidal long-acting oral anti-inflammatory drugs that do not have the adverse effects of steroids or of selective leukotriene inhibitors.

SUMMARY The present invention is directed to a medicament for use in a method of treating asthma or COPD in a human patient in need of such treatment, wherein the lungs of said patient are affected by a respiratory Haemophilus influenza, Streptococcus pneumoniae or Moraxella catarrhalis infection, or a combination thereof, said method comprising administering to said patient, by oral inhalation, a therapeutically effective amount of said medicament, wherein said medicament is norketotifen, an R or S atropisomer, a prodrug or a pharmaceutically acceptable salt thereof, wherein the prodrug is as defined in claim 1.

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods for treatment of the human body by therapy, according to the present invention.

In one aspect, not falling within the scope of the claimed subject-matter, a method of treating a respiratory disorder in a human patient in need of such treatment comprises orally administering to the human patient in need thereof a therapeutically effective amount of norketotifen, an isomer, a prodrug, or a pharmaceutically acceptable salt thereof, wherein a daily loading dosage of the norketotifen, isomer, prodrug or pharmaceutically acceptable salt thereof is administered for about 3 to 7 days, and on the first day following completion of the about 3 to 7 days of the daily loading dosage, a daily maintenance dosage of the norketotifen, isomer, prodrug or pharmaceutically acceptable salt thereof is administered for at least 10 days, wherein the daily maintenance dosage is equal to or less than one half, equal to or less than one third, or equal to or less than one quarter of the daily loading dosage.

A method, not falling within the scope of the claimed subject-matter, of treating asthma or COPD in a human patient in need of such treatment comprises administering by oral inhalation a therapeutically effective amount of norketotifen, an isomer, a prodrug or a pharmaceutically acceptable salt thereof to the patient suffering from asthma or COPD, wherein the lungs of said patient are affected by a respiratory bacterial, fungal or mold infection, and wherein the therapeutically effective amount of the norketotifen, isomer, prodrug or pharmaceutically acceptable salt thereof provides a pulmonary concentration that is equal to or greater than the minimum inhibitory concentration (MIC) value for the bacteria, fungus or mold.

A method, not falling within the scope of the claimed subject-matter, of treating an airways disorder in a human patient in need of such treatment comprises administering to the nasal passages of the human patient in need thereof a therapeutically effective amount of norketotifen, an isomer, a prodrug or a pharmaceutically acceptable salt thereof to reduce a symptom of the airways disorder, wherein the airways disorder is non-allergic rhinitis, vasomotor rhinitis, nonallergic rhinitis with eosinophilia, chronic rhinitis, laryngitis, sinusitis, or nasopharyngitis.

### DETAILED DESCRIPTION

Described herein are studies showing that norketotifen is even more potent than the well-known steroid prednisone /prednisolone in the treatment of respiratory disorders, but importantly, norketotifen is the first potent anti-inflammatory pulmonary drug to be free from the adverse immune-suppressive effects of the steroids. Thus, norketotifen can be used as a potent anti-inflammatory drug without adverse immune-suppressant activities.

It has now also been found that norketotifen expresses anti-microbial effects against several types of bacteria, fungi and mold that commonly infect the lungs of patients suffering from pulmonary disorders such as asthma and COPD and also of patients suffering from inflammatory airways disorders. Prior to the present disclosure, it had not been shown that orally administered norketotifen has antimicrobial pulmonary effects. As shown herein, inhaled norketotifen is undoubtedly reaching and exceeding the pulmonary concentrations needed for antimicrobial efficacy.

The methods described herein relate to methods of treating respiratory disorders including airways and pulmonary disorders by administering norketotifen, a R or S atropisomer, a prodrug, or a pharmaceutically acceptable salt thereof, orally, by oral inhalation or by nasal inhalation. In an aspect, the compound is RS-norketotifen. Prior to the present disclosure, the respiratory therapeutic efficacy of norketotifen administered orally and by inhalation and had not been confirmed. Since norketotifen is a low-toxicity drug, the oral doses of norketotifen can be high during the initial loading phase after starting oral or inhalation treatment with norketotifen in patients with pulmonary conditions. The oral loading dose period may last up to a week or more and will be followed by maintenance treatment. Daily dose/doses and the duration of the loading dose treatment of individual patients will be determined by the medical doctor or the care-giver of the patient.

Respiratory disorders include both airways and pulmonary disorders. Airways disorders include such disorders as rhinitis, allergic rhinitis, non-allergic rhinitis, vasomotor rhinitis, nonallergic rhinitis with eosinophil syndrome (NARES), chronic rhinitis, atrophic rhinitis, senile rhinitis, cerebral spinal fluid leak, sinusitis, laryngitis, acute bronchitis, cough, chronic bronchitis, and nasopharyngitis.

Rhinitis is an inflammation of the nasal mucosal membranes, which is often expressed as swelling of the nasal membranes, nasal congestion, rhinorrhea, with various and well-known symptoms, such as itching, sneezing, and purulence.

Allergic rhinitis is an inflammation of the nasal mucosal membranes, caused by the interaction of allergens with IgE type antibodies, which in turn leads to the release of pro-inflammatory cells and the degranulation of such cells.

The remaining forms of rhinitis are referred as non-allergic rhinitis (NAR). NAR includes vasomotor rhinitis (also called non-allergic rhinopathy), nonallergic rhinitis with eosinophilia (NARES), chronic rhinitis, atrophic rhinitis, senile rhinitis, and cerebrospinal fluid leak.

Vasomotor rhinitis (VMR) is a chronic inflammatory condition in which intermittent vascular engorgement of the nasal mucous membranes leads to watery rhinorrhea and repeated sneezing. The etiology is uncertain, but no allergy can be identified. A dry atmosphere appears to aggravate this condition. An estimated 14 million Americans suffer from vasomotor rhinitis with a worldwide prevalence approaching 320 million. Norketotifen, administered by oral or intranasal administration is of therapeutic use because of the long-acting anti-inflammatory activity, the pulmonary distribution after systemic administration, and the lack of immune-suppressive adverse effects of this drug.

] Nonallergic rhinitis with eosinophilitis (NARES) is an inflammation of the nasal mucous membranes and is expressed as edema and vasodilatation and often rhinorrhea. Nasal cytology analysis demonstrates more than 20 percent eosinophils. Anosmia (decreased sense of smell) is a prominent feature. NARES is a self-perpetuating chronic eosinophilic nasal inflammation with development of nasal polyposis. Mast cells may also play an important role in NARES. While VMR is the most common subtype of NAR, NARES makes up the majority of the remaining diagnoses. Norketotifen, administered orally or nasally, for example, is expected to potently decrease the edema through the anti-inflammatory activity of the drug, while norketotifen administered by nasal spray devices, for example, will deliver norketotifen in concentrations high enough for the drug to also offer antimicrobial effects in addition to the anti-inflammatory and antipruritic effects of the drug.

Chronic rhinitis is an inflammation of the nasal mucosa and is often a prolongation of subacute inflammatory rhinitis or infectious rhinitis. Chronic rhinitis may be caused by prolonged exposure to dry climate or to dry air in airplanes. Chronic rhinitis is often expressed as nasal obstruction, purulent rhinorrhea and/or nasal bleedings.

The initial trigger of atrophic rhinitis is a bacterial infection of the nasal lining, which leads to chronic inflammation of the nasal mucosa. The chronic condition is preferably treated with long-acting anti-inflammatory drug. Since norketotifen is not an immune-suppressant drug and it has advantages over the immune-suppressant corticosteroids. It is not preferred to treat patients with infections with immune-suppressive drugs.

Senile rhinitis is rhinitis in the geriatric population. Senile rhinitis is an inflammation of the nasal mucosa and is characterized by congestion, rhinorrhea, itching of the nose, postnasal drip, sneezing, crusting within the nose, cough, olfactory loss and/or nasal dryness. Non-allergic rhinitis, including senile rhinitis, is often treated with oral steroids, such as prednisolone. Norketotifen can replace steroids in the treatment of NARs and norketotifen has been shown to be equivalent to prednisolone or to be more efficacious as an anti-inflammatory drug than prednisolone. The use of norketotifen will avoid all steroidal adverse effects, not only adverse immune suppression.

Cerebral spinal fluid leaks are a type of non-allergic rhinitis that are typically corrected surgically.

Sinusitis is a common disease with an annual prevalence of about 10 percent of the people in USA and 30 percent of the people in Europe. Sinusitis is defined as an inflammation of the mucous membrane that lines the paranasal sinuses, most often caused by viral, bacterial or fungal infections or allergic reactions. Acute sinusitis is resolved in less than 30 days, and subacute sinusitis is usually resolved in 30 to 90 days, while recurrent (or chronic) sinusitis is expressed with episodes with about one or two symptom-free weeks between such episodes. Acute and subacute sinusitis can be treated with corticosteroids or with the non-immuno-suppressive drug norketotifen that will decrease the inflammatory pressure in the sinuses and thereby improve sinus drainage and decrease the pain for the patients. Recurrent (chronic) sinusitis may require surgery to improve sinus drainage.

Laryngitis is an inflammatory disease of the larynx, which usually is due to overuse or to viral infection. The yearly incidence of chronic laryngitis has been calculated as about 3.5 cases per 1,000 people. Steroidal anti-inflammatory drugs are typically prescribed, and norketotifen is believed to become a drug of choice because it is a potent and long-acting and non-steroidal anti-inflammatory drug and may therefore be preferred as an orally inhaled remedy for laryngitis. Gargling with a syrup containing norketotifen may be a preferred route to administer high concentration of norketotifen directly to the biophase. Norketotifen may be co-administered with a proton pump inhibitor for the treatment of laryngitis.

Acute bronchitis and acute bronchitis with cough are caused by inflammation. In addition to coughing, the symptoms of acute bronchitis include wheezing, shortness of breath, and chest pain. Bronchitis can be acute or chronic. Acute bronchitis usually has a cough that lasts for about three weeks and is in most cases caused by a viral infection. The treatment of acute bronchitis with cough is typically focused on decreasing the inflammation, and corticosteroids are often prescribed. Norketotifen is a non-immunosuppressant alternative to corticosteroids and a significantly more potent and longer acting anti-inflammatory alternative to current therapy with self-medicated over-the-counter NSAIDs. The prevalence of acute bronchitis is high with about five percent of adults are affected yearly and about six percent of children have at least one episode of acute bronchitis a year.

Chronic bronchitis is defined as a productive cough that lasts for three months or more per year for at least two years. Typically, inflammatory cell infiltrates are found in the airways walls of patients suffering from chronic bronchitis, which, in addition to accumulation and degranulation of pro-inflammatory eosinophils, will provide beneficial effects of norketotifen in these patients.

Nasopharyngitis is an inflammatory condition. Nasopharyngitis is most often caused by viral infections, typically rhinovirus, or by human coronavirus or influenza viruses. The symptoms of nasopharyngitis, e.g. cough, sore throat, nasal congestion, afasic conditions and edema of the airways are inflammatory disorders from the virus infection(s) or from concomitant bacterial infections. Locally applied steroids have beneficial anti-inflammatory effects, but are potent immune-suppressant drugs and shall therefore not be used to treat the symptoms of infectious diseases. Nasal decongestants cause nasal vasoconstriction after nasal insufflation, however these drugs, which usually are adrenergic alpha-receptor agonists, develop strong tachyphylaxis after repeated use and can actually cause local vasodilation and worsening of the nasal congestion, called rhinitis medicamentosa. Self-medication by individuals suffering from common cold most often include NSAIDS, such as ibuprofen or aspirin. What is needed by nasopharyngitis patients is an anti-inflammatory drug that does not cause immunosuppression but will express long-acting and potent anti-inflammatory activity such as norketotifen.

In the treatment of airways disorders, norketotifen expresses potent anti-inflammatory effects without causing adverse local or systemic immune-suppression.

As used herein, pulmonary disorders are characterized by decreased airflow, and also include inflammation. Pulmonary disorders include asthma and COPD.

Asthma is a common long-term inflammatory disease of the airways. It is characterized by variable and recurring symptoms, reversible airflow obstruction, and easily triggered bronchospasms. Symptoms include episodes of wheezing, coughing, chest tightness, and shortness of breath. Asthma can be classified as atopic and non-atopic. The symptoms of asthma can sometimes be prevented by avoiding triggers, such as allergens and irritants and by use of inhaled anti-inflammatory drugs, such as acute anti-inflammatory drugs or long-acting drugs that prevents the inflammatory symptoms of asthma.

Chronic obstructive pulmonary disease (COPD) is characterized by obstructive inflammation causing inhibited airflow and poor breathing. The older term "chronic bronchitis" is used to define a productive and recurrent cough, while the term "emphysema" is still used and refers to the existence of air in the pulmonary tissues. In contrast to asthma, the airflow reduction does not improve much with the use of bronchodilators in COPD patients. Tobacco smoking is the primary risk factor for development of COPD.

Eosinophilic asthma has been defined as asthma with sputum cell counts from 1 percent to 3 percent. Eosinophilic COPD can be defined as COPD with a cut-off eosinophil level in blood of at least 2 percent. Patients suffering from nonallergic rhinitis with eosinophilia syndrome demonstrate nasal cytology analysis with more than 20 percent eosinophils. Norketotifen is particularly potent in inhibiting pulmonary eosinophil accumulation in a well-known laboratory animal model (Example 1; Table 1) and it has therefore been concluded that norketotifen inhibit eosinophil accumulation also in human patients.

In humans, both asthma and COPD are currently treated with anti-inflammatory corticosteroids, inhaled bronchodilators and most often, combinations thereof. To decrease systemic adverse effects and to shorten the onset time of the medication, corticosteroids are administered directly to the lungs by inhalation devices, such as for example hydrofluoroalkane (HFA) inhalers, metered dose inhalers (MDI), dry powder inhalers (DPI) and nebulizers. Adverse effects from use of inhalation devices are few although thrush (an oral yeast infections) and hoarseness may occur. Thrush is treated with oral antifungal medications and hoarseness is usually treated by rinsing the mouth (gargling).

An advantage of using norketotifen for the treatment of asthma and COPD is that the risk for adverse systemic immune-suppression is decreased. Unlike corticosteroids which must be dosed at the lowest dose possible, norketotifen may be used in higher oral doses than possible for steroids, particularly since oral administration of norketotifen has now been found to results in surprisingly high pulmonary concentrations of the drug.

In addition, the use of norketotifen can avoid the side effects of corticosteroids such as adrenal gland atrophy; cataracts; facial hair growth; glaucoma; growth retardation in children; headache; high blood pressure; increased blood glucose and loss of diabetes control; loss of potassium; menstrual irregularity; muscle weakness; obesity; osteoporosis; puffiness of the face (moon face); slow wound healing; sodium and fluid retention causing edema and weight gain; thinning and easy bruising of the skin; ulcers in the stomach and duodenum; and others.

As an alternative to corticosteroids, montelukast (Singulair^{®}, Merck) is an orally administered leukotriene inhibitor. Montelukast is preferred by patients who suffer from exercise-induced asthma. Montelukast has been linked to depression and suicidal thoughts, even in children. Use of norketotifen rather than leukotriene inhibitors can also avoid the side effects of leukotriene inhibitors.

What is needed for the treatment of respiratory disorders are non-steroidal long-acting oral anti-inflammatory drugs that do not have the adverse effects of steroids or of leukotriene inhibitors, such as Montelukast.

In the treatment of respiratory disorders, norketotifen does not cause adverse immune suppression which is contrary to the steroids. Thus, norketotifen may be used in higher doses and higher concentrations and for longer periods of time than possible for steroids.

The methods described herein relate to the treatment of respiratory disorders, such as pulmonary disorders and airways disorders, in human patients, by oral dosing, by oral inhalation, or by nasal inhalation of norketotifen or a R or S atropisomer or a prodrug or a pharmaceutically acceptable salt thereof. In an aspect, the compound is RS-norketotifen. Prior to the present disclosure, the respiratory/pulmonary therapeutic efficacy of orally administered norketotifen, for example, had not been reported. Since norketotifen is a low-toxicity drug, the oral doses of norketotifen can be high during the initial loading phase and reduced during a maintenance phase.

Of particular note, norketotifen differs from the glucocorticoids, since norketotifen, after oral administration, is rapidly absorbed and is surprisingly preferentially distributed to the lungs, where the concentration of RS-norketotifen can reach concentrations that are 100 times higher than the plasma concentration. This finding (see Table 3) is particularly surprising since it is contrary to current teaching that pulmonary drug concentrations cannot exceed the plasma concentration.

In addition to oral administration, the RS-norketotifen can be administered to the nasal passages using nasal drops or nasal sprays or by oral inhalation devices such as for example metered dose inhalers, dry powder inhalers, HFA inhalers and nebulizers using doses needed and as often as needed by the patient and selected by his/her physician or caregiver.

As used herein, norketotifen refers to norketotifen, a R or S atropisomer, a prodrug, or a pharmaceutically acceptable salt thereof. RS-norketotifen refers to racemic norketotifen. In an aspect, the compound is RS-norketotifen hydrogen fumarate.

Norketotifen is an achiral molecule, but has two atropisomers, S-norketotifen and R-norketotifen, as has previously been described by Aberg et al. in U.S. Patent Nos. 7,226,934 and 7,557,128.

As explained in U.S. Patents 7,226,934 and 7,557,128, norketotifen had significant sedative effects when studied in an art-accepted mouse model of sedation, and further, the sedative effects were attributed to the R-isomer. It was thus proposed that only the S-isomer could be administered without sedative side effects. It has later been found that orally administered RS-norketotifen is free from sedative side effects in dogs (U.S. Patent No. 8,557,846) and in humans (U.S. Patent Nos. 9,138,431 and 9,345,697). Therefore, unlike for ketotifen, no dose-limiting sedative adverse effects are expected for norketotifen, even after high oral doses of norketotifen.

Norketotifen can be made using methods known in the art, as described in U.S. Patent No. 3,682,930.

Prodrugs of norketotifen include N-substituted hydroxyalkyl or carboxyalkyloxyalkyl analogs thereof. Such molecules are described in U.S. Patent No. 6,297,683. Prodrugs of norketotifen include molecules of the formula: 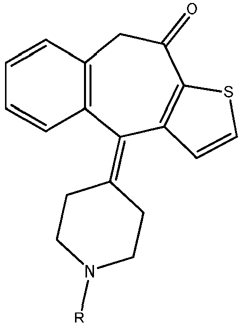 wherein R is hydroxy-C₂-C₆ alkyl or carboxy-C₁-C₆alkoxy-C₁-C₆alkyl. Additional prodrugs include substituents at the 8-position, the 10-position and/or in the 12 to 17 positions and/or inclusion of substituents on various positions on the piperidine ring.

As used herein, the terms "pharmaceutically acceptable salts" or "a pharmaceutically acceptable salt thereof" refer to norketotifen salts, which have been prepared from pharmaceutically acceptable non-toxic acids. Exemplary pharmaceutically acceptable acid as for the compound of the present invention include acetic, benzenesulfonic (besylate), benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pathothenic, phosphoric, p-toluenesulfonic, succinic, sulfuric, tartaric, and the like. The hydrochloride salt and the hydrogen fumarate salt are particularly preferred.

Embodiments disclosed herein provide for the oral administration of norketotifen or its pharmaceutically acceptable acid addition salts to human patients in need of treatment for respiratory disorders. Norketotifen is particularly well suited for the treatment of respiratory disorders, such as for example non-allergic rhinitis (NAR), asthma and COPD, since norketotifen is a potent and long-acting non-steroidal anti-inflammatory drug.

Oral administration of norketotifen to treat respiratory disorders, for approximately 1 week or less (loading dose) can then be followed by a lower maintenance dose, which at the discretion of the caregiver can be continued for 1 week, 10 days, weeks, months or years. This is particularly important because conditions such as asthma and COPD are most often chronic conditions, requiring treatment for weeks, months, or years. Thus, norketotifen will preferably and initially be administered once or twice daily for up to one week, which is the loading dose, followed by a lower, maintenance dose of norketotifen once or twice daily or less frequently than once daily for one week, several weeks, one month, several months, one year or several years.

More specifically, in a study in dogs, (Table 2), once daily orally administered norketotifen was found to accumulate in the lungs, and the concentration in the lungs was 70 times higher than in the plasma. In a similar study in rats, the pulmonary concentration of norketotifen was 100 times higher in lungs than in plasma (Table 3). It is quite surprising that the concentration of norketotifen in the lungs is higher than the plasma drug concentration because it is generally believed in the art that pulmonary concentrations of drugs are not higher than the corresponding plasma-drug concentrations due to the lack of known transporters between the blood and the lungs.

A method, not falling within the scope of the claimed subject-matter, of treating a respiratory disorder in a human patient in need of such treatment comprises orally administering to the human patient in need thereof a therapeutically effective amount of norketotifen, an isomer, a prodrug, or a pharmaceutically acceptable salt thereof, wherein oral administration of a daily loading dosage is followed by administration of a daily maintenance dosage that is less than the loading dosage.

In a specific non-claimed aspect, a method of treating a respiratory disorder in a human patient in need of such treatment comprises orally administering to the human patient in need thereof a therapeutically effective amount of norketotifen, an isomer, a prodrug, or a pharmaceutically acceptable salt thereof, wherein a daily loading dosage of the norketotifen, isomer, prodrug or pharmaceutically acceptable salt thereof is administered for about 3 to 7 days, and on the first day following completion of the about 3 to 7 days of the daily loading dosage, a daily maintenance dosage of the norketotifen, isomer, prodrug or pharmaceutically acceptable salt thereof is administered for at least 10 days, wherein the daily maintenance dosage is equal to or less than one half, equal to or less than one third, or equal to or less than one quarter of the daily loading dosage. In an aspect, it is possible for the maintenance dose to be administered once every other day or less frequently.

In an aspect, the norketotifen is administered in the form of a tablet, a capsule, or a syrup. In another aspect, the method further comprises further administering a second therapeutically active agent, specifically a long-acting muscarinic antagonist, a long-acting beta receptor agonist, or a combination thereof.

In an aspect, the oral daily loading dosage is from about 1 mg to about 30 mg of the norketotifen, R or S atropisomer, prodrug or pharmaceutically acceptable salt thereof, calculated as norketotifen free base and administered one or more times daily. For example, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 mg of norketotifen, R or S atropisomer, prodrug or pharmaceutically acceptable salt thereof can be administered as the loading dosage.

In an aspect, the oral daily maintenance dosage is maintenance dosage is from about 0.5 mg to about 20 mg of norketotifen, R or S atropisomer, prodrug or pharmaceutically acceptable salt thereof, calculated as norketotifen free base, and administered one or more times daily. For example, about 0.5, 1, 2, 3, 4, 5, 6, 7, 8, 9, 10, 11, 12, 13, 14, 15, 16, 17, 18, 19 or 20 mg of norketotifen, R or S atropisomer, prodrug or pharmaceutically acceptable salt thereof can be administered as the maintenance dosage.

In an aspect, the respiratory disorder for treatment with oral norketotifen is a pulmonary disorder such as asthma or COPD.

In another aspect, the respiratory disorder for treatment with oral norketotifen is an airways disorder such as non-allergic rhinitis, vasomotor rhinitis, non-allergic rhinitis with eosinophil syndrome, chronic rhinitis, senile rhinitis, sinusitis, laryngitis, acute bronchitis, acute bronchitis with cough, chronic bronchitis, or nasopharyngitis.

In general, due to the severity of the disease, oral loading doses for treatment of COPD, for example, can be two or three times higher and the loading dose period can be up to twice as long as the loading dose period for asthma patients.

In an aspect, the oral administration of norketotifen avoids adverse effects associated with chronic administration of other potent anti-inflammatory drugs, such as corticosteroids, calcineurin inhibitors, phosphodiesterase-4 inhibitors, Janus kinase inhibitors and anti-inflammatory monoclonal antibodies, all of which cause adverse immune system suppression.

In addition to its anti-inflammatory properties, norketotifen has antimicrobial activity and is expected to inhibit the pulmonary growth of microorganisms such as fungi, specifically molds, and bacteria including *Malassezia sp, Trichophyton sp., Candida albicans,* and *Staphylococcus sp. Staphylococcus sp.* bacterial infections of the lungs are not uncommon in human patients and a clinical study demonstrated mortality of 32 percent of these patients, despite antibiotic treatment of the pulmonary infections. Pulmonary infections with *Candida sp* are well-known and pulmonary *Malassezia sp.* infections are also well-known. Thus, the respiratory disorders treatable with norketotifen in accordance with the dosing regimens described herein can include bacterial and fungal pulmonary infections.

In another aspect, disclosed herein is a method of treating asthma or COPD in a human patient in need of such treatment, comprising administering by oral inhalation a therapeutically effective amount of norketotifen, a R or S atropisomer, a prodrug or a pharmaceutically acceptable salt thereof to the patient suffering from asthma or COPD, wherein the lungs of said patient are affected by a respiratory bacterial, fungal or mold infection. In an aspect, the therapeutically effective amount of the norketotifen, or S atropisomer, prodrug or pharmaceutically acceptable salt thereof provides a pulmonary concentration that is equal to or greater than the minimum inhibitory concentration (MIC) value for the bacteria, fungus or mold. Table 6 provides exemplary MIC values.

In general, a free pulmonary concentration that is equal to or greater than the minimum inhibitory concentration (MIC) value for the bacteria, fungi or mold cannot be achieved by oral, e.g., systemic, administration. Only oral inhalation of norketotifen can provide concentrations of norketotifen that are high enough to kill microbes in the lungs. Administering norketotifen by oral inhalation (for example by a dry powder inhaler) can save the lives of COPD patients by killing the microbes that cause life-threatening pulmonary infections. In addition, norketotifen prevents inflammation that accompanies infection.

In another aspect, the therapeutically effective amount of the norketotifen, R or S atropisomer, prodrug or pharmaceutically acceptable salt thereof reduces or eliminates a symptom of the respiratory bacterial, fungal or mold infection in the patient. Exemplary symptoms of respiratory infection include cough with phlegm, stabbing chest pain, shortness of breath, difficult breathing, wheezing, yellow or green colored mucus, fever, chills, throat pain, sinus drainage or congestion, headache, and combinations thereof.

Pulmonary infections can be diagnosed by listening for abnormal sounds in the lungs when a patient is breathing. In addition, X-ray and CT scans can be helpful to diagnose bacterial pneumonia in the lower respiratory tract. The respiratory infection can be diagnosed by medical imaging (chest x-ray or CT scan), spirometry, pulse oximetry, mucus culture, throat swab, complete blood count, blood culture, or a combination of the foregoing.

In an aspect, administration by inhalation is performed using a dry powder inhaler, a metered dose inhaler, an HFA inhaler, a nebulizer, or a digital inhaler.

In an aspect, the therapeutically effective amount of inhaled norketotifen, or a R or S atropisomer, or a prodrug or a pharmaceutically acceptable salt thereof, for providing therapeutically effective pulmonary concentration will depend on the disease of the patient (asthma or COPD) and the severity of concomitant pulmonary microbial infections. While any suitable inhaler may be used, patients suffering from severe pulmonary infections may prefer a dry powder inhaler that can deliver from 10 µg and up to 500 µg of micronized norketotifen per actuation. The inhaled dose of norketotifen in severely sick patients may consist of up to six or more daily actuations from a high-capacity DPI device.

Because norketotifen is not a penicillin and because norketotifen accumulates in the lungs, it is expected to be effective in the treatment of infections with penicillin-resistant bacteria in the lungs. Exemplary bacteria include methicillin-resistant *Staphylococcus aureus* (MRSA), vancomycin-resistant *Enterococcus* (VRE), drug-resistant *Streptococcus pneumoniae* (DRSP) and multi-drug resistant *Mycobacterium tuberculosis* (MDR TB). It is understandable by those skilled in the art that norketotifen can be combined with other anti-microbial drugs.

In addition to inhibiting the growth of bacteria, norketotifen has been found to inhibit the growth of fungi, particularly the mold *Trichophyton* sp. such as *Trichophyton rubric,* which can be found in the lungs of subjects with asthma and COPD. Thus, in an aspect, norketotifen can be administered to subjects in need of treatment for *Trichophyton* asthma or *Trichophyton* COPD. In an aspect, the subject demonstrates fungal sensitization demonstrated by an increase in serum IgE specific to *Trichophyton* sp. In an aspect, the subject demonstrates fungal sensitization demonstrated by a positive skin test to *Trichophyton* sp. antigens. It is also expected that RS-norketotifen will be effective against pulmonary infections caused by *Alternaria* sp., *Cladosporium* sp. and *Penicillium* sp.

In another aspect, and built on the surprising and potent anti-mold activity of norketotifen (Example 6), methods of treating Trichophyton asthma or Trichophyton COPD in a patient in need of such treatment and comprising oral and/or inhaled administration to the patient in need thereof a therapeutically effective amount of racemic or isomeric norketotifen or a pharmaceutically acceptable salt thereof.

In another aspect, a method of treating airways mycosis in a patient in need of such treatment comprises administering to the patient in need thereof a therapeutically effective amount of racemic or isomeric norketotifen or a pharmaceutically acceptable salt thereof by oral and/or inhalation routes.

Norketotifen can also be used to treat subjects in need of treatment for allergic bronchopulmonary mycosis (ABPM), which develops mainly in patients with asthma via types I and III hypersensitivity reactions to filamentous fungi. *Aspergillus* spp., especially *Aspergillus fumigatus,* is the major causative fungus. *Aspergillus fumigatus* is typically found in the soil, however, in certain people, the immune system reacts to *Aspergillus fumigatus* antigens in the lungs. In an aspect, the subject expresses fungal sensitization demonstrated by an increase in total serum IgE and/or, the presence of IgE and IgG antibodies specific to causative fungi such as *Aspergillus fumigatus.* In another aspect, the subject demonstrates fungal sensitization demonstrated by a positive skin test to fungal antigens.

The inventors have also unexpectedly found that norketotifen inhibits muscarinic M-3 receptors. Inhibitors of muscarinic M-3 receptors inhibit constriction of bronchi and bronchioles, which may become important in the treatment of asthma and emphysema, a form of COPD.

New drugs for COPD, such as Trelega Ellipta^{®}, GSK, contain a steroidal anti-inflammatory (such as fluticasone) and a LABA (long-acting beta receptor agonist, such as vilanterol) and a LAMA (long-acting muscarinic antagonist), such as umeclidine, glycopyrrolate, or tiotropium.

A LABA may also be added to the combination therapy of NK for selected patients with severe bronchospasms. An example of a LABA to be added is vilanterol.

In another aspect, a method of treating an airways disorder in a human patient in need of such treatment comprises administering to the nasal passages of the human patient in need thereof a therapeutically effective amount of norketotifen, a R or S atropisomer, a prodrug or a pharmaceutically acceptable salt thereof to reduce a symptom of the airways disorder, wherein the airways disorder is non-allergic rhinitis, vasomotor rhinitis, nonallergic rhinitis with eosinophilia, chronic rhinitis, laryngitis, sinusitis, or nasopharyngitis.

Exemplary symptoms of non-allergic rhinitis, vasomotor rhinitis, nonallergic rhinitis with eosinophilia, chronic rhinitis, sinusitis, or nasopharyngitis include inflammation of the nasal membranes with nasal congestion, rhinorrhea, itching, sneezing, purulence, increased body temperature and/or nasal bleeding, and combinations thereof.

In an aspect, administration to the nasal passages comprises nasal insufflation, nasal inhalation or administration by nose drops.

In an aspect, the therapeutically effective amount of norketotifen, R or S atropisomer, prodrug or pharmaceutically acceptable salt thereof for administration for relief of the symptoms is about 10 µg to about 1 mg per actuation, calculated as norketotifen free base.

In an aspect, a method of treating respiratory disorder in a human patient in need of such treatment comprises orally administering to the human patient in need thereof a therapeutically effective amount of norketotifen and administering a bronchodilating adrenergic beta-2 receptor agonist such as formoterol. Formoterol, for example, can be administered by inhalation. In an aspect, the norketotifen and the bronchodilating adrenergic beta-2 receptor agonist are the only drugs administered to the subject to treat the respiratory disorder. Exemplary doses are as described above.

In another aspect, a method of treating a respiratory disorder in a human patient in need of such treatment comprises orally administering to the human patient in need thereof an anti-inflammatory effective amount of norketotifen, wherein the norketotifen is the only anti-inflammatory agent administered to the subject to treat the respiratory disorder. Exemplary doses are as described above.

The embodiments disclosed herein also provide pharmaceutical compositions, which comprise norketotifen, formulated together with one or more pharmaceutically acceptable carriers.

Pharmaceutical compositions for oral administration of solid dosage forms include capsules, tablets and liquid dosage forms. In solid dosage forms, the active compound may be mixed with one or more pharmaceutically acceptable excipients or carriers (such as for example sodium citrate, dicalcium phosphate), fillers or extenders (such as for example starch, lactose, sucrose, glucose, mannitol, silicic acid), binders (such as for example alginates, carboxymethylcellulose, gelatin, polyvinylpyrrolidone, sucrose, acacia), humectants (such as for example glycerol), solution retarding agents (such as for example paraffin), disintegrating agents (such as for example agar-agar, calcium carbonate, starch, alginic acid, silicates, sodium carbonate), absorption accelerators (such as for example quaternary ammonium compounds), wetting agents (such as for example cetyl alcohol, glycerol monostearate), absorbents (such as for example kaolin, bentonite clay), lubricating agents (such as for example talc, calcium stearate, magnesium stearate, polyethylene glycols, sodium lauryl sulfate), and/or other excipients, such as for example buffering agents. Solid forms of capsules, granules, pills, and tablets can have coatings and/or shells (such as for example enteric coatings) known in the art. The compositions may also be designed to release the active ingredient(s) in a certain part of the gastrointestinal tract or in a controlled release, slow-release or in a delayed-release manner. The active compound(s) can also be microencapsulated with one or more of the above-mentioned excipients or other suitable excipients.

Liquid dosage forms for oral administration of norketotifen include pharmaceutically acceptable emulsions, solutions, suspensions, syrups and elixirs. The liquid dosage form may also contain commonly known diluents (such as for example water, other solvents, solubilizing agents), emulsifiers, such as for example ethanol, isopropyl alcohol, ethyl carbonate, ethyl acetate, benzyl alcohol, benzyl benzoate, propylene glycol, butylene glycol, dimethyl formamide, oils, oleic acid, glycerol, polyethylene glycols, sorbitan fatty esters, and mixtures thereof.

The actual dosage levels of active ingredients in the pharmaceutical compositions disclosed herein may be varied so as to obtain the desired therapeutic effect. Thus, the amount of drug used varies and will depend on factors such as the administration form, the severity of the disease, the frequency of dosing, and other circumstances (such as general health, body weight, age, etc.) known to the patient, the caretaker of the patient and/or the caring physician.

The therapeutically effective oral doses of norketotifen useful for treating human patients with pulmonary conditions will be determined by the caring physician and are generally 0.5 mg to 50 mg (calculated as norketotifen free base), dosed orally as the free base or as a salt, such as for example the hydrochloride or mesylate salts or the hydrogen fumarate salt, once, twice or more times daily. In one embodiment, the treatment is once daily dosing. The therapeutically effective dose may be administered less than once daily, such as for example one to six times weekly, wherein administration is over at least a one-week period, as determined by the patient, the caretaker of the patient and/or the caring physician.

The embodiments disclosed herein provide methods for treatment of disorders of the lungs in patients with norketotifen, while avoiding the sedating side effects of ketotifen and other benzocycloheptathiophene compounds, and while avoiding the adverse immune-suppressant effects of corticosteroids (see Examples 4, 5) and other potent anti-inflammatory compounds. The embodiments also provide treatment of pulmonary microbial disorders in human patients. Administering to the patient in need of such treatment, can consist of effective amounts of norketotifen free base or a pharmaceutically acceptable salt thereof, at a dosing frequency to be determined by the individual human patient, the caretaker of the patient and/or the caring physician. In one embodiment, frequency of the therapy is one or more doses/day of norketotifen during the first week/weeks of therapy and one or two daily doses during the following long-term maintenance therapy. The dosing under long-term maintenance therapy may be reduced to one single weekly dose. In one embodiment, frequency of the therapy is one or more doses/day of norketotifen during the initial loading-dose period and during the following maintenance dosing period. The dosing during the long-term maintenance therapy may be reduced to less than once daily, such as for example one single weekly dose.

In addition to the use of norketotifen as single-drug medication in human patients, embodiments disclosed herein also provide methods for co-administration of norketotifen with at least one drug of one of the following classes: bronchodilating agents, antibacterial agents, antifungal agents, antiviral agents, vitamin D or vitamin D analogs, cyclooxygenase inhibitors, leukotriene antagonists, lipoxygenase inhibitors, selective inhibitors of one or more cytokines, such as for example kinase inhibitors and immunomodulators, such as for example cyclosporine. The co-administration may be temporary or may be chronically used in the patient. Norketotifen and the co-administered drug can be administered to the patient separately or can be co-formulated with norketotifen for oral, parenteral, pulmonary or dermal administration. Thus, as an example, norketotifen can be administered orally and the co-administered drug may also be administered orally or by inhalation. Furthermore, norketotifen and the co-administered drug may not be administered simultaneously. Thus, as an example, norketotifen may be administered orally once daily, or once weekly, while a co-administered adrenergic agonist may have to be administered orally or pulmonary (by inhalation), once or more times daily.

When used for the treatment of pulmonary disorders, such as for example asthma or COPD, norketotifen can be combined with a therapeutically active dose of a bronchodilating drug and the bronchodilating drug can independently be administered by inhalation, nasal, parenteral, topical, transdermal, rectal, sublingual or oral administration. Common bronchodilating drugs are short-acting adrenergic beta-receptor agonists, long-acting adrenergic beta-receptor agonists, anticholinergic drugs and also methylxanthines, such as for example theophyllin. The adrenergic beta-receptor agonist can, but will not necessarily be, selected from the group consisting of albuterol (salbutamol), terbutaline, fenoterol, formoterol, and salmeterol and the optically and therapeutically active isomers of adrenergic beta-receptor agonists. Examples of anticholinergic bronchodilators are tiopropium and ipratropium and a well-known bronchodilating methylxanthin is theophylline. Since both bronchial inflammation and broncoconstriction are hallmarks of asthma and COPD, it is advantageous that norketotifen is expressing both anti-inflammatory and broncho-dilating activities. In cases where additional bronchodilatation is needed, norketotifen can be supplemented with additional broncho-dilating drugs, such as an adrenergic beta-receptor agonist or an additional antimuscarinic M-3 drug.

There is a known inhibition of adrenergic beta-receptor down-regulation by corticosteroidal drugs. Norketotifen inhibits such down-regulation of adrenergic beta-receptors. In one embodiment, the method further comprises co-administration of norketotifen and an adrenergic beta-receptor agonist, wherein norketotifen inhibits said adrenergic beta-receptor down-regulation.

Common bronchodilators that are currently used together with inhaled steroids are long-acting adrenergic beta-2 receptor agonists (LABAs), long-acting muscarinic M-3 antagonists (LAMAs) or combinations thereof. The two market leaders for the treatment of asthma are combinations of the anti-inflammatory corticosteroid fluticasone and the long-acting adrenergic beta-receptor agonist salmeterol (Advair^{®}, Glaxo), and the combination of the anti-inflammatory corticosteroid budesonide and long-acting adrenergic beta-receptor agonist formoterol (Symbicort^{®}, Astra-Zenica). LABAs and LAMAs may be combined with norketotifen.

The invention is further illustrated by the following non-limiting examples.

### EXAMPLES

### Example 1. Pulmonary anti-inflammatory effects in vivo after oral or parenteral doses of norketotifen

The migration of eosinophils to the lungs of asthma- and COPD-patients is well known. Various pulmonary cells express adhesion molecules, to which eosinophils bind with great avidity, leading to accumulation, degranulation and release of toxic eosinophil cationic protein (ECP), toxic eosinophil peroxidase (EPO) and eosinophil-derived neurotoxin, thereby causing severe inflammatory responses of the lung tissues.

### METHODS

Male Sprague-Dawley rats, 400-600 g, were used in these studies.

The Main Study described here used subcutaneous administration of norketotifen and ketotifen by Alzet^{®} osmotic pumps. The rats were administered either RS-norketotifen hydrogen fumarate (1.0 mg/kg/day) or RS-ketotifen hydrogen fumarate (1.0 mg/kg/day) or saline. After five days of sc infusions of test articles and the vehicle, the animals were injected, i.p. with 10 µg PAF (platelet aggregating factor) in 0.25% bovine serum albumin (BSA) in saline. The subcutaneous infusions of test articles or vehicle were continued for another twenty-four hours and the animals were then sacrificed by intraperitoneal injections of a barbiturate. The tracheae of the euthanized animals were exposed and cannulated and aliquots (6 x 10 ml) of a Tyrode solution were successively introduced into the lungs and aspirated by gentle compression of the thorax. The total recovery of lung fluid was usually above 80%. The cell suspensions were concentrated by low speed centrifugation (200 g for 10 min) and the resulting cell pellet was re-suspended in 1 ml of a Tyrode solution. Total cell counts were performed after dilution in Turks fluid, fixation in methanol and Leishman staining.

The Main Study shown here used norketotifen, ketotifen, and saline (control) after continuous dosing with Alzet^{®} Osmotic pumps. The administration of PAF increased the pulmonary eosinophil counts to 250 percent, as shown in Table 1. Subcutaneous Alzet^{®} dosing of ketotifen, 1 mg/kg/day for 6 days, reduced the PAF-induced eosinophilia to almost non-PAF levels and the sc dosing of norketotifen 1.0 mg/kg/day, for six days, had a supra-maximal effect, blocking the response to PAF completely and further reduced the eosinophil counts by 26 % below the baseline (Control) level, as shown in Table 1.

**Table 1. Inhibition of pulmonary eosinophil accumulation after continuous subcutaneous dosing of test articles, 1 mg/kg/day for six days**

| TEST ARTICLE (1 mg/kg/day sc for 6 days) | N | PAF-induced esosinophilia (% ± SEM) |
|---|---|---|
| BSA + saline (Control) | 8 | 100 ± 9 |
| PAF + BSA + saline (Control) | 8 | 250 ± 4 |
| PAF + BSA + Ketotifen HF | 8 | 110 ± 3 |
| PAF + BSA + Norketotifen HF | 10 | 74 ± 4 |

| | | |
|---|---|---|
| BSA = bovine serum albumin; PAF = platelet aggregating factor HF = hydrogen fumarate (salt) BSA = Bovine Serum Albumin PAF = platelet aggregating factor | | |

### CONCLUSIONS

Both ketotifen and norketotifen reduced PAF-induced eosinophilia. Norketotifen was significantly more potent as a PAF-inhibitor than ketotifen. Thus, after subcutaneous dosing of the test articles (Table 1), norketotifen completely inhibited all the effects of PAF and further reduced the eosinophil counts to a level that was 26 percent below the saline control level.

In a follow-up study, it was found that the plasma concentrations of norketotifen in the Main Studies were very low: 4.0 ± 0.24 ng/ml after sc infusion of RS-norketotifen for 5 days, indicating a surprisingly high potency of norketotifen against pulmonary PAF-induced eosinophilia.

Most of the activity of ketotifen in this study is believed to be due to the activity of norketotifen since ketotifen is readily metabolized to norketotifen in rodents.

As known by those skilled in pharmacology, an oral dose of 1 mg/kg to rats corresponds to a Human Equivalent Dose (HED) of 0.16 mg/kg or a total human dose of approximately 1 mg to a human, weighing 60 kilograms. Similarly, an oral dose of 3 mg/kg to rats, corresponds to a total dose of approximately 3 mg to a human, weighing 60 kg.

Based on the results from this and similar studies and the important roles played by eosinophils in asthma and COPD and other respiratory diseases, it was concluded that norketotifen will be particularly effective as treatment for severe eosinophilic inflammatory diseases, such as eosinophilic asthma, eosinophilic COPD and non-allergic rhinitis with eosinophilia.

### Example 2. Lung / Plasma Distribution of Norketotifen (NK) In Dogs

PURPOSE: To determine concentrations of NK in lungs and plasma after oral dosing of NK to beagle dogs.

### METHODS

One group (N=3) of female Beagle dogs (8 to 10 kg / 24 to 28 months) were administered racemic norketotifen hydrogen fumarate (RS-NK-HF) 11.46 mg/kg/day for 4 consecutive days. (11.46 mg of NK-HF is equal to = 8.0 mg of the free base (RS-NK-FB).

Plasma samples were collected daily on Days 2, 3 and 4. The last blood samples were taken four hours after dosing on Day 4. Lung and plasma concentrations of norketotifen were determined using a qualified LC-MS-MS analytical method. LLQ was <0.5 ng/ml. Immediately after blood collection on Day 4, all animals were euthanized with an overdose of pentobarbital, administered intravenously, followed by exsanguination. Lung tissue samples (approximately 1 g each) were collected from each animal from the left and right pulmonary lobes, samples were trimmed and dried of any access blood using absorbent paper, weighed and then kept in conical tubes frozen on dry ice and stored at -80°C pending analysis. Six samples from each animal (3 samples per side) were analyzed. The data is shown in Table 2.

**Table 2. Day 4 Plasma and Lung Concentrations of NK in dogs at 2 hours after oral doses, corresponding to 8.0 mg/kg/day of NK-FB for four days**

| Animal Number | Day | Post-dose Lung Concentration (ng/g) | Post-dose Plasma Concentration (ng/mL) | Lung / Plasma Ratio in Dogs |
|---|---|---|---|---|
| 001 | 4 | 23590 | 301 | 78.4 |
| 002 | 4 | 79546 | 1651 | 48.2 |
| 003 | 4 | 61093 | 733 | 83.3 |
| Mean ± SEM | | | | 70.0 ± 11.0 |

CONCLUSIONS: The results from this study in three dogs demonstrated that the concentration of norketotifen was on an average 70 times higher in the lungs than in the plasma.

### Example 3. Lung / Plasma Distribution of Norketotifen In Rats

PURPOSE: Due to the surprising results from the Example 2 study in dogs, the decision was made to repeat the Example 2 study using a different species and more power.

### METHODS

Groups of male SPD rats (200 - 230 g) were administered racemic norketotifen hydrogen Fumarate 20 mg/kg/day (= 14.4 mg/kg/day of the free base) for 10 consecutive days. Lung and plasma samples were collected and prepared and the concentrations of RS-norketotifen were determined using a carefully qualified LC-MS-MS analytical method. LLQ (Lowest Level of Quantification) was <0.5 ng/ml. The results are provided in Table 3.

**Table 3. Concentrations of NK free in lungs and plasma of rats during and after once daily oral doses of NK-FB, corresponding to 14.4 mg/kg free base for 10 days**

| Study Day | Lung Concentration (ng/g) | Plasma Concentration (ng/mL) | Lung/Plasma Ratio in Rats |
|---|---|---|---|
| Dose Day 4 | 23612 | 178 | 133 |
| Dose Day 7 | 25355 | 276 | 92 |
| Last dose. Day 10 | 18171 | 229 | 79 |
| Last dose + 24 hrs | 890 | 8.3 | 107 |
| Mean ± SEM | | | 103 ± 11.6 |

### CONCLUSION

There was a significant accumulation of norketotifen in lungs of rats with about 100 times (103 times) higher concentration of norketotifen in lungs than in plasma. The results from this study confirm the surprising results from the Example 2 study.

The high dose of norketotifen used in the Example 3 study was selected to correspond to the use of a once-a-day loading dose of norketotifen for 10 days. The results from this study demonstrate no advantage of extending the use of a loading dose in excess of seven days.

The accumulation of norketotifen in the lungs is surprising and there is no known explanation for pulmonary accumulation that can be applied to the present results.

It was concluded that the results from the drug distribution studies in dogs and rats indicate that oral administration of norketotifen may replace inhalation administration of steroidal drugs for patients suffering from respiratory diseases.

### Example 4: Studies on Immunosuppression

Objective: This study was performed to evaluate possible immunosuppressant activity of RS-norketotifen.

Methods: Male BALB/c mice, 20-24 grams, 7-12 weeks, were used. The sensitization dosing (Day 1) consisted of painting the ventral portion of the pre-shaved abdominal skin with 0.1ml of 3.0 % oxazolone in 70 % ethanol. Test articles were administered orally in 1.0 % methyl-cellulose, starting on Day 1 and continuing for 10 consecutive days. The challenge dosing consisted of the application of 0.05 ml of 3.0% oxazolone in 70% ethanol on both sides of the outer pinna of the right ear. The thickness of both ears of all animals was measured with a calliper before the challenge dose and 24 hours after the administration of the challenge dose (Day 10). The ear thickness of untreated mice was 0.19 to 0.20 mm. Oxazolone is not a pro-inflammatory compound and the results shown here demonstrate immuno-suppressive effects, not anti-inflammatory activity. The results are shown in Table 5.

**Table 4. Immunosuppression in vivo in male BalB/c mice**

| Test Article | N | Dose/day (mg/kg) | Increase in ear thickness (mm) | Increase in ear thickness (%) | Immunosuppression (%) | Statistical significance (P-value) |
|---|---|---|---|---|---|---|
| Control | 16 | --- | 0.153 | 100.0 | 0 | --- |
| NK | 16 | 10.0 | 0.147 | 96.1 | 3.9 | ≥ 0.25 (NS) |
| NK | 16 | 30.0 | 0.140 | 91.5 | 8.5 | ≥ 0.25 (NS) |
| PRED | 8 | 0.3 | 0.108 | 70.6 | 29.4 | < 0.02 |
| PRED | 8 | 1.0 | 0.084 | 55.0 | 45.0 | < 0.0001 |
| PRED | 8 | 3.0 | 0.076 | 49.7 | 50.3 | < 0.0001 |

| | | | | | | |
|---|---|---|---|---|---|---|
| NK = RS-norketotifen. PRED = prednisolone. NS = no statistical significance. "Ear thickness" refers to the difference in ear thickness between Day 9 (before the challenge dose) and Day 10 (24 hours after the challenge dose). | | | | | | |

Conclusions: Daily oral dosing of RS-norketotifen 10 or 30 mg/kg for 10 days to mice did not induce immuno-suppressant activity. The reference compound, prednisolone, expressed potent and dose-dependent immune suppression at doses that were 10 times lower than the concentrations of norketotifen

Moreover, parameters examined during repeat-dose toxicology studies revealed no signs of immune system suppression after assessment of: (1) Hematology Indicators; (2) Clinical Chemistry Indicators; (3) Histopathology indicators; (4) Organ and Body Weight indicators.

Justification of the model: The classic oxazolone test method was used with minor modifications. This method has been used for over 40 years in numerous drug development projects. Oxazolone does not cause inflammation and the results shown here refer to immune-suppressive effects

### Example 5. Anti-inflammatory effects of norketotifen and prednisolone.

Objective: To compare anti-inflammatory effects of oral doses of norketotifen (NK) and prednisolone (PRED).

Methods: The effects of NK, PRED and the vehicle (5% PEG in water) were tested orally in CD-1 female mice. Under the test protocol, 20 µl of 1.0% croton oil in acetone was applied topically to both ears of three groups of mice, each group consisting of 6 mice. No attempt was made to remove the croton oil solution since the solvent evaporated within 30 seconds. The oral doses of NK and PRED were 10 mg/kg (10 ml/kg) bodyweight for both compounds and were administered 60 min before the croton oil applications. Vehicle-treated mice were dosed 10 ml/kg of water, containing 5% polyethylene glycol (PEG), which was also the solvent for the test articles. Ear thickness was measured with an electronic caliper and mean ear thickness (N=12; right + left ears) were calculated and compared with the thickness of ears from vehicle-treated mice (Student's t-test). As shown in Table 5, NK, but not PRED, expressed statistically significant anti-inflammatory effects after oral dosing.

**Table 5. Effects of single-dose NK and single-dose PRED on croton oil-induced ear swelling**

| Time after croton oil, min | Average ear thickness | | |
|---|---|---|---|
| | Vehicle | NK | PRED |
| 0 (predose) | 0.21 ± 0.02 | 0.21 ± 0.02 | 0.22 ± 0.01 |
| 90 | 0.29 ± 0.04 | 0.24 ± 0.02 *** | 0.28 ± 0.01 |
| 120 | 0.28 ± 0.02 | 0.25 ± 0.02 *** | 0.27 ± 0.01 |

| | | | |
|---|---|---|---|
| N = 12 ears /group. NK = norketotifen PRED = prednisolone | | | |

Conclusions: NK expressed statistically significant anti-inflammatory effects when compared with the vehicle control group. PRED did not express anti-inflammatory effect in this study, probably due to less potency or to slower onset of activity, or both.

### Example 6. Antimicrobial activity of norketotifen

Microorganisms such as the fungi *Aspergillus fumigatus, Malassezia globosa* and *Candida albicans,* the molds *Tricophyton rubrum* and *Trichophyton interdigitale* and the bacteria *Staphylococcus aureus, Staphylococcus intermedius, Klebsiella pneumoniae, Haemophilus influenza* and *Streptococcus pneumoniae* grow in human lungs, particularly in immune-compromised patients in whom the growth of such microorganisms in the lungs is enhanced by steroids and other immune-suppressive drugs.

Objective The current studies were designed to study antimicrobial activities of norketotifen (NK) against selected toxic microbes that are commonly found in the lungs of immune-compromised patients.

Methods Samples of NK were challenged with between 1.0×10⁵ to 1.0×10⁶ cfu/ml of microorganisms. The organisms were inoculated in centrifuge tubes containing 10 mL of NK solutions and 1.0 mL samples were aliquoted from each centrifuge tube weekly. The logarithmic reductions of microorganism concentrations were determined by the plate count method by diluting in DEB (D/E neutralizing Broth) from 10⁻¹ to 10⁻⁴ for fungi, mold and bacteria. Bacterial plates were poured with SCDA (Soybean Casein Digest Agar) and incubated at 32.5±2.5°C for 3-5 days. -- The water solubility of NK about 2% (20 mg/ml). As shown in Table 6, the MICs (Minimum Inhibitory Concentrations) for NK were read from the plates.

**Table 6. Antimicrobial effects of NK -- MIC-values (mg/ml for norketotifen)**

| | Fungi: MIC | | | Mold: MIC | | Bacteria MIC | | | | | | |
|---|---|---|---|---|---|---|---|---|---|---|---|---|
| | Af | Mp | Ca | Tr | Ti | Sa | MRSA | Si | Kp | Hi | Sp | Mc |
| NK (mg/mL) | 4.0 | 1.0 | 1.0 | 0.5 | 0.5 | 0.5 | 2.0 | 0.25 | 0.5 | 4.0 | 0.5 | 0.25 |

| | | | | | | | | | | | | |
|---|---|---|---|---|---|---|---|---|---|---|---|---|
| Fungi: Af = Aspergillus fumigatus. Mp = Malassezia pachydermatis. Ca = Candida albicans Mold: Tr = Trichophyton rubrum. Ti = Trichophyton interdigitale. Bacteria: Sa = Staphylococcus aureus. MRSA = Methicillin-resistant S. aureus. Si = Staphylococcus intermedius. Kp = Klebsiella pnemoniae. Hi = Haemophilus influenza. Sp = Streptococcus pneumoniae, Mc = Moraxella catarrhalis. MIC refers to lethal concentrations of NK. Those skilled in microbiology realize that static concentrations, such as bacteriostatic and fungistatic concentrations of NK are lower than the lethal (MIC) concentrations. | | | | | | | | | | | | |

The MIC-concentrations for norketotifen shown in Table 6 are high and will not be reached after oral administration of norketotifen. These concentrations of norketotifen will be reached in the lungs only after administered of norketotifen to the patient by use of inhalation devices, such as for example dry powder inhalers (DPI).

Microbial infections play an important role as aeliologic factors in COPD. Thus, it has been established that bacteria cause up to 50 percent of acute exacerbations in COPD-patients. Bacteria also play a major role in asthma exacerbations. Chronic microbial persistence in lungs and airwayss is not innocuous, but cause progression of pulmonary inflammation and airways obstruction in asthma patients.

### Example 7. Effects of Norketotifen on Muscarinic M-3 Receptor Binding

The purpose of this study was to test NK in a muscarinic M-3 receptor binding assay.

Background: It is known to those skilled in the art that acetylcholine is binding to smooth muscle muscarinic receptors. Binding of acetylcholine to muscarinic M-3 receptors in the airwayss is causing airwayss constriction and inhibition of the muscarinic receptors will cause bronchial smooth muscle dilatation.

Methodology: The current study used human M-3 (hM3) receptor and NK hydrogen fumarate was the antagonist in the current studies. The study used NK in duplicates at five dose-levels from 1.0E-08M to 1.0E-04M.

Results: As calculated from the mean values Ki (M) was calculated to be 2.2E-06M of norketotifen free base.

Conclusions: The pulmonary distribution of NK shall be kept in mind (Example 2, above) since the concentration of NK in lungs was found to be 70 to 100 times higher than the plasma concentration of NK. It is therefore currently believed that no LAMA (Long-acting- Anti-Muscarinic Agent) will be needed by most patients when NK is administered orally to individuals with asthma or COPD.

### Example 8: Plasma Level of RS-norketotifen in the eosinophil accumulation study

Plasma samples were not taken during the eosinophil accumulation studies (Example 1), as no validated analytical method for assessing the concentration of norketotifen in rat plasma was available at the time the studies were conducted. When a validated analytical method became available the following study was performed.

### METHODOLOGY

Ten male Sprague-Dawley rats, weighing 410 - 430 grams, were implanted subcutaneously with Durect Alzet^{®} Osmotic Pumps that had been prepared for continuous dosing of racemic norketotifen hydrogen fumarate at a rate of 1.0 mg/kg/day. All animals were closely monitored during the study period for any signs of ill health. Observations were recorded daily using the current LabCat^{®} In-Life module and all animals appeared normal throughout the study.

Blood was sampled at two time points. One blood sample was taken from the orbital sinus on Day 6 (after sc infusion of norketotifen for 5 days) and another sample was taken approximately 24 hours later from the abdominal aorta (Day 7; after sc infusion of norketotifen for 6 days) during barbiturate anesthesia and prior to euthanasia. Blood samples were collected in lithium heparin tubes, centrifuged at approximately 4°C, 2000 rpm, for 20 minutes, and the plasma obtained from each sample was recovered and stored frozen (-80°C) pending analysis.

All plasma samples were analyzed for S-norketotifen and R-norketotifen, using validated analytical methodology. Total concentrations of norketotifen were calculated as Concentration of S-norketotifen + Concentration of R-norketotifen.

### RESULTS

The total plasma concentration of norketotifen (venous; orbital sinus) was 4.0 ± 0.24 ng/ml on Day 6 (after sc infusion of RS-norketotifen for 5 days) and 5.6 ± 0.50 ng/ml on Day 7 (arterial; abdominal aorta); after sc infusion of RS-norketotifen for 6 days.

### CONCLUSIONS

The steady-state plasma levels of NK were in the single-digit ng/ml range and it was concluded that norketotifen completely inhibited the severe PAF-induced eosinophilia at plasma concentrations of norketotifen in the single-digit nanogram/ml range.

As known by those skilled in pharmacology, an oral dose of 1 mg/kg to rats corresponds to a Human Equivalent Dose (HED) of 0.16 mg/kg or a total human dose of approximately 1 mg to a human, weighing 60 kilograms. Similarly, an oral dose of 3 mg/kg of a test article to rats, corresponds to a total dose of approximately 3 mg to a human, weighing 60 kg.

### Example 9: Exemplary oral dosage formulation.

To make tablets, NK is blended with lactose and cellulose until a uniform blend is formed. The blue lake is added and further blended. Finally, the calcium stearate is blended in, and the resulting mixture is compressed into tablets using for example a 9/32-inch (7 mm) shallow concave punch. Tablets of other strengths may be prepared by altering the ratio of active ingredient to the excipients or to the final weight of the tablet. Those skilled in the art realize that formulations can also be administered to the patient in the form of for example a capsule, a cream, an ointment or a liquid formulation. Both norketotifen salts and norketotifen free base can be formulated as tablets.

**Table 7. Tablet formulations**

| Ingredient | Amount per tablet | Amount per batch |
|---|---|---|
| Norketotifen (NK) | 8 mg | 800 g |
| Microcrystalline cellulose | 24 mg | 2400 g |
| Lactose | 56 mg | 5600 g |
| Calcium stearate | 1.4 mg | 140 g |
| FD&C Blue #1 Lake | 0.03 mg | 3 g |

### Example 10: Inhalation dosage forms

In an aspect, a dosage form for inhalation is a nebulizer. Exemplary nebulizer devices include the Respimat^{®}, Soft Mist^{™} Inhaler (Boehringer Ingelheim), the AERx^{®} Pulmonary Delivery System (Aradigm Corp.), and the PARI LC Plus^{®} Reusable Nebulizer (Pari GmbH). An exemplary composition for use in a nebulizer inhaler comprises an isotonic aqueous solution comprising from about 0.05 µg/mL to about 10 mg/mL of norketotifen. In one aspect, such a solution has a pH of about 3.5-6.

Alternatively, a composition comprising the active agent(s)/active ingredient(s) may be administered by inhalation using a dry powder inhaler (DPI). DPIs typically administer the active agent as a free-flowing powder that is dispersed in a subject's air-stream during inspiration. In order to achieve a free flowing powder, the active agent(s)/active ingredient(s) is typically formulated with a suitable excipient such as lactose, starch, mannitol, dextrose, polylactic acid, polylactide-co-glycolide, or combinations thereof. Typically, the active agent(s)/active ingredient(s) is micronized and combined with an excipient to form a blend suitable for inhalation. Accordingly, in one aspect, the active agent(s)/active ingredient(s) is in micronized form. For example, a representative composition for use in a DPI (dry powder inhaler) comprises dry lactose having a particle size between about 1 µm and about 100 µm (e.g., dry milled lactose) and micronized particles of the active agent. Such a dry powder formulation can be made, for example, by combining lactose with the active agent and then dry blending the components. Alternatively, if desired, the active agent can be formulated without an excipient. The composition is then typically loaded into a DPI, or into inhalation cartridges or capsules for use with a DPI. DPIs are well known to those of ordinary skill in the art, and many such devices are commercially available, with representative devices including Aerolizer^{®} (Novartis), Airmax^{™} (IVAX), ClickHaler^{®} (Innovata Biomed), Diskhaler^{®} (GlaxoSmithKline), Diskus^{®} or Accuhaler^{™} (GlaxoSmithKline), Easyhaler^{®} (Orion Pharma), Eclipse^{®} (Aventis), FlowCaps^{®} (Hovione), HandiHaler^{®} (Boehringer Ingelheim), Pulvinal^{®} (Chiesi), Rotahaler^{®} (GlaxoSmithKline), SkyeHale^{™} or Certihaler^{®} (SkyePharma), Twisthaler^{®} (Schering-Plough), Turbuhaler^{®} (AstraZeneca), Ultrahaler^{®} (Aventis), and the like.

Alternatively, the composition comprising the active agent may be administered by inhalation using a metered-dose inhaler (MDI). Such MDIs typically discharge a measured amount of the active agent using compressed propellant gas. Metered-dose formulations thus typically comprise a solution or suspension of the active agent in a liquefied propellant, such as a chlorofluorocarbon such as CCl₃F or a hydrofluoroalkane (HFA) such as 1,1,1,2-tetrafluoroethane (HFA 134a) and 1,1,1,2,3,3,3-heptafluoro-n-propane (HFA 227), although HFAs are generally preferred due to concerns about chlorofluorocarbons affecting the ozone layer. Additional optional components of HFA formulations include co-solvents, such as ethanol or pentane, and surfactants, such as sorbitan trioleate, oleic acid, lecithin, and glycerin. A representative composition for use in an MDI comprises from about 0.01-5 wt % of active agent; from about 0-20 wt % ethanol; and from about 0-5 wt % surfactant; with the remainder being an HFA propellant. Such compositions are typically prepared by adding a chilled or pressurized hydrofluoroalkane to a suitable container containing the active agent, ethanol (if present) and the surfactant (if present). To prepare a suspension, the active agent is micronized and then combined with the propellant. The formulation is then loaded into an aerosol canister, which forms a portion of the MDI. MDIs are well known to those of ordinary skill in the art, and many such devices are commercially available, with representative devices including AeroBid^{®} Inhaler System (Forest Pharmaceuticals), Atrovent^{®} Inhalation Aerosol (Boehringer Ingelheim), and the like. Alternatively, a suspension formulation can be prepared by spray drying a coating of surfactant on micronized particles of the active agent.

### Example 11: Intranasal dosage forms

Intranasal dosage forms can be formulated in an aerosol form, spray, mist or in the form of drops. Intranasal compositions can include a mucoadhesive agent, a solubilizer, a preservative, a flavoring agent, a vehicle, and combinations thereof.

Examples of mucoadhesive agent include, but are not limited to polyacrylic polymers like carbopols, polycarbophil, carboxymethylcellulose or its pharmaceutically acceptable salt, microcrystalline cellulose, hydroxyethylcellulose, hydroxypropyl cellulose, hydroxypropylmethyl cellulose (i.e., hypromellose), methylcellulose, poloxamers, pectin, xanthan gums, alginates, gelatin alone or in any combination thereof. Nasal compositions can contain about 0.05 to about 5% w/v of a mucoadhesive agent.

Examples of solubilizers (or crystal growth inhibitors) include, but are not limited to d-alpha tocopheryl polyethylene glycol 1000 succinate (Vitamin E TPGS), macrogol (15)-hydroxystearate (Solutol HS 15), polyoxyethylene-polyoxypropylene copolymer (Poloxamer, Pluronic, such as poloxamer 188), PEO-PLLA diblock copolymer, PEG-PLGA-PEG triblock, copolymer, cyclodextrins, hydroxypropyl betadex, polyoxyethylene castor oil derivatives, povidone, sulfobutylether-b-cyclodextrin, tricaprylin, triolein, glyceryl monostearate, sorbitan esters (sorbitan fatty acid esters), polyoxyethylene fatty acid esters, polysorbate 80, polysorbate 20 or macrogol-15-hydroxysterate. Nasal compositions can contain from about 0.2 to about 10.0% w/v of a solubilizer.

Examples of preservatives include benzalkonium chloride, sodium benzoate, methyl, ethyl, propyl or butyl paraben, benzyl alcohol, phenylethyl alcohol, benzethonium chloride, chlorobutanol, potassium sorbate or combination thereof. Nasal compositions can contain from about 0.01 to about 1% w/v of a preservative. Liquid compositions containing norketotifen may not need an added preservative since the antimicrobial activity of the norketotifen molecule can make the formulations self-preserving, which is advantageous since preservatives, such as benzalkonium chloride are toxic entities.

Exemplary flavoring agents include flavor anise, flavor apple, flavor apricot, flavor banana, flavor buttermint, flavor citrus, flavor orange, flavor menthol mint, flavor mint, flavor peppermint, flavor spearmint, alone or in any combinations thereof. Nasal compositions disclosed may contain from about 0.01% w/v to about 0.5% w/v of a flavoring agent.

Examples of vehicles include, but are not limited to, saline, water, dextrose or combinations thereof. The pH of compositions described herein may be about 3.0 to about 7.4 and all values in between.

As used herein, the terms "pharmaceutically acceptable salts" or "a pharmaceutically acceptable salt thereof" refer to norketotifen salts, which have been prepared from pharmaceutically acceptable non-toxic acids. Exemplary pharmaceutically acceptable acid as for the compound of the present invention include acetic, benzenesulfonic (besylate), benzoic, camphorsulfonic, citric, ethanesulfonic, fumaric, gluconic, glutamic, hydrobromic, hydrogen fumaric, hydrochloric, isethionic, lactic, maleic, malic, mandelic, methanesulfonic, mucic, nitric, pamoic, pathothenic, phosphoric, p-toluenesulfonic, succinic, sulfuric, tartaric, and the like. The hydrochloride salt and the hydrogen fumarate salt are particularly preferred.

The term "antimicrobial" as used herein refers to antibacterial, antifungal and anti-mold activities or effects.

The term "patient" as used herein refers to human patients and canine patients.

The use of the terms "a" and "an" and "the" and similar referents (especially in the context of the following claims) are to be construed to cover both the singular and the plural, unless otherwise indicated herein or clearly contradicted by context. The terms first, second etc. as used herein are not meant to denote any particular ordering, but simply for convenience to denote a plurality of, for example, layers. The terms "comprising", "having", "including", and "containing" are to be construed as open-ended terms (i.e., meaning "including, but not limited to") unless otherwise noted.

As used herein, when referring to dosage amount, the term "about" includes amounts to ± 10% of the recited value.

As used herein, the term "chronic administration" is defined as three or more consecutive days of administration. Acute administration of norketotifen refers to a single administration of the drug.

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the invention as used herein.

## Claims

1. A medicament for use in a method of treating asthma or COPD in a human patient in need of such treatment, wherein the lungs of said patient are affected by a respiratory Haemophilus influenza, Streptococcus pneumoniae or Moraxella catarrhalis infection, or a combination thereof, said method comprising administering to said patient, by oral inhalation, a therapeutically effective amount of said medicament, wherein said medicament is norketotifen, an R or S atropisomer, a prodrug or a pharmaceutically acceptable salt thereof,
wherein the prodrug is 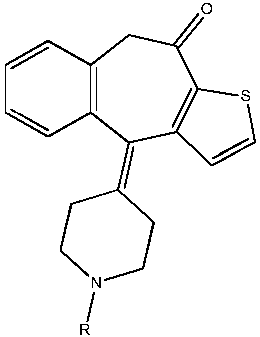 wherein R is hydroxy-C₂-C₆ alkyl or carboxy-C₁-C₆alkoxy-C₁-C₆alkyl.

2. The medicament for use according to claim 1, wherein the patient is in need of treatment for an acute exacerbation of chronic obstructive pulmonary disorder (COPD).

3. The medicament for use according to claim 1, wherein in said method the therapeutically effective amount reduces or eliminates a symptom of the respiratory infection in the patient.

4. The medicament for use according to claim 3, wherein the symptom of respiratory infection is selected from cough with phlegm, stabbing chest pain, shortness of breath, difficult breathing, wheezing, yellow or green colored mucus, fever, chills, throat pain, sinus drainage or congestion, headache, or a combination thereof.

5. The medicament for use according to claim 1, wherein in said method the respiratory infection is diagnosed by medical imaging, spirometry, pulse oximetry, mucus culture, throat swab, complete blood count, blood culture, or a combination of the foregoing.

## Patentansprüche

1. Arzneimittel zur Verwendung in einem Verfahren zur Behandlung von Asthma oder COPD bei einem menschlichen Patienten, der eine solche Behandlung benötigt, wobei die Lungen des Patienten von einer Haemophilus-Influenza der Atemwege, Streptococcus pneumoniae oder Moraxella catarrhalis Infektion oder einer Kombination davon betroffen sind, wobei das Verfahren das Verabreichen einer therapeutisch wirksamen Menge des Arzneimittels durch orale Inhalation an den Patienten umfasst, wobei das Arzneimittel Norketotifen, ein R- oder S-Atropisomer, eine Pro-Drug oder ein pharmazeutisch annehmbares Salz davon ist,
wobei die Pro-Drug Folgendes ist: 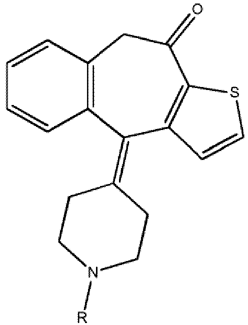 wobei R Hydroxy-C₂-C₆-Alkyl oder Carboxy-C₁-C₆-Alkoxy-C₁-C₆-Alkyl ist.

2. Arzneimittel zur Verwendung nach Anspruch 1, wobei der Patient eine Behandlung für eine akute Verschlimmerung von chronisch obstruktiver Lungenerkrankung (COPD) benötigt.

3. Arzneimittel zur Verwendung nach Anspruch 1, wobei in dem Verfahren die therapeutisch wirksame Menge ein Symptom der Infektion der Atemwege bei dem Patienten vermindert oder beseitigt.

4. Arzneimittel zur Verwendung nach Anspruch 3, wobei das Symptom der Infektion der Atemwege ausgewählt ist aus Husten mit Schleim, stechenden Schmerzen in der Brust, Kurzatmigkeit, Atembeschwerden, Keuchen, gelb oder grün gefärbtem Schleim, Fieber, Schüttelfrost, Halsschmerzen, Ausfluss oder Verstopfung der Nebenhöhlen, Kopfschmerzen oder einer Kombination davon.

5. Arzneimittel zur Verwendung nach Anspruch 1, wobei in dem Verfahren die Infektion der Atemwege durch medizinische Bildgebung, Spirometrie, Pulsoxymetrie, Sputumkultur, Rachenabstrich, vollständiges Blutbild, Blutkultur oder eine Kombination des Vorhergehenden diagnostiziert wird.

## Revendications

1. Médicament destiné à l'utilisation dans une méthode de traitement de l'asthme ou de la BPCO chez un patient humain ayant besoin d'un tel traitement, dans lequel les poumons dudit patient sont affectés par une infection respiratoire à Haemophilus Influenza, Streptococcus pneumoniae ou Moraxella catarrhalis, ou une combinaison de ceux-ci, ladite méthode comprenant l'administration audit patient, par inhalation orale, d'une quantité thérapeutiquement efficace dudit médicament, ledit médicament étant le norkétotifène, un atropisomère R ou S, un promédicament ou un sel pharmaceutiquement acceptable de celui-ci,
le promédicament étant 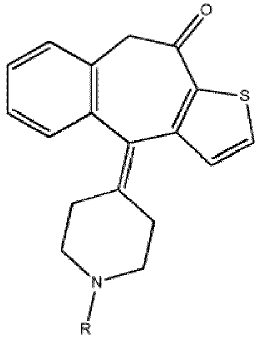
où R est un hydroxyalkyle en C₂-C₆ ou carboxy(alcoxy en C₁-C₆) -alkyle en C₁-C₆.

2. Médicament destiné à l'utilisation selon la revendication 1, dans lequel le patient a besoin d'un traitement pour une exacerbation aiguë de la bronchopneumopathie chronique obstructive (BPCO).

3. Médicament destiné à l'utilisation selon la revendication 1, dans lequel, dans ladite méthode, la quantité thérapeutiquement efficace réduit ou élimine un symptôme de l'infection respiratoire chez le patient.

4. Médicament destiné à l'utilisation selon la revendication 3, dans lequel le symptôme de l'infection respiratoire est choisi parmi la toux avec glaires, la douleur thoracique lancinante, le souffle court, la difficulté à respirer, la respiration sifflante, un mucus de couleur jaune ou verte, la fièvre, les frissons, le mal de gorge, un écoulement ou une congestion des sinus, les maux de tête, ou une combinaison de ceux-ci.

5. Médicament destiné à l'utilisation selon la revendication 1, dans lequel, dans ladite méthode, l'infection respiratoire est diagnostiquée par imagerie médicale, spirométrie, oxymétrie de pouls, culture de mucus, prélèvement pharyngé, hémogramme complet, hémoculture, ou une combinaison de ce qui précède.
